# DEMANDE DE BREVET EUROPEEN

(11) **EP 1 563 885 A1**
(43) Date de publication de la demande: **17.08.2005**
(21) Numéro de dépôt: 05290112.1
(22) Date de dépôt: 19.01.2005
(51) Int. Cl.: B01D 11/02, A61K 7/06

(54) **Procédé de préparation d'une composition cosmétique pour le traitement des matières kératiniques à partir de fluide sous pression et de substances végétales non colorantes.**

(30) Priorité: 29.01.2004 FR 0400847
(71) Demandeur: L'OREAL, 75008 Paris (FR)
(72) Inventeur: De la Mettrie, Roland, 78110 Le Vesinet (FR); Müller, Rainer, 76344 Leopoldshafen (DE)
(74) Mandataire: Casalonga, Axel

(57) **Abrégé**

L'invention concerne un procédé de préparation d'une composition pour le traitement cosmétique des matières kératiniques. Ce procédé comprend une étape de percolation de fluide comprenant au moins de la vapeur d'eau, sous une pression d'au moins 3 bars au travers d'au moins une substance végétale non colorante sous forme solide ou pâteuse.

L'invention concerne aussi un procédé de traitement des matières kératiniques mettant en oeuvre ladite composition.

## Description

La présente invention a pour objet un procédé de préparation d'une composition destinée au traitement cosmétique des matières kératiniques telles que la peau et les fibres kératiniques, par exemple, les cheveux, ainsi qu'un procédé de traitement cosmétique des matières kératiniques à partir de cette composition.

En cosmétique, on cherche toujours à améliorer les propriétés cosmétiques des matières kératiniques. Il est ainsi connu d'utiliser dans des compositions pour application topique des agents de traitement provenant de végétaux, permettant de lutter contre les agressions extérieures comme la pollution et le rayonnement ultraviolet, de lutter contre les dommages des fibres kératiniques ou de la peau, notamment les signes du vieillissement de la peau, d'hydrater et de nourrir les fibres kératiniques et la peau et d'apporter à la peau et aux autres matières kératiniques traitées par ces compositions, tous les bienfaits liés à ces agents de traitement.

En particulier, ces agents de traitement peuvent être utilisés pour le traitement de la peau, des cheveux, des cils, des lèvres et des ongles, par exemple pour nourrir ou hydrater la peau, la protéger des agents extérieurs, éliminer les peaux mortes, diminuer les rides et ridules ainsi que les taches colorées (défaut de pigmentation ou taches apparaissant avec l'age en particulier sur les mains, le cou ou le visage).

L'utilisation d'agents de traitement provenant de végétaux est connue en cosmétique, notamment pour l'obtention de propriétés adoucissantes, anti-inflammatoires, antiprurigineuses, antiseptiques, antisudorales, astringentes, calmantes, cicatrisantes, raffermissantes, tonifiantes, parfumantes, moussantes, odorantes, absorbantes, épaississantes, antioxydantes, émulsifiantes, photoprotectrices et/ou texturantes. Les formulations contenant de telles substances actives sont généralement obtenues par différents procédés tels que macération, digestion, décoction, infusion ou lixiviation.

Cependant, les compositions de traitement cosmétique contenant de tels agents de traitement sont généralement des compositions aqueuses dans lesquelles lesdits agents doivent être solubilisés. Le manque de solubilité de ces composés diminue le caractère traitant de ces compositions. En outre, ce critère de solubilité réduit le nombre d'agents de traitement provenant de végétaux que l'on peut utiliser pour le traitement cosmétique des matières kératiniques, ainsi que le nombre de compositions les contenant que l'on peut offrir aux consommateurs.

Par ailleurs, de nombreux composés naturels présentent l'inconvénient d'être instables vis-à-vis de l'eau ou d'autres composés organiques ou minéraux utilisés couramment en cosmétique, à savoir ils subissent une dégradation par un mécanisme d'hydrolyse ou de réaction avec lesdits composés organiques ou minéraux, ce qui conduit à des compositions cosmétiques peu efficaces et/ou d'aspect, d'odeur et/ou toucher inacceptables pour le consommateur, en quelques jours.

La Demanderesse a découvert de manière surprenante qu'en utilisant un nouveau procédé de préparation d'une composition de traitement cosmétique des matières kératiniques, on pouvait obtenir en un temps très court, inférieur à 2 minutes, des compositions plus ou moins concentrées en agent(s) de traitement provenant de végétaux, selon le besoin, et notamment sans conservateur, permettant de surmonter les problèmes de solubilité et de stabilité exposés ci-dessus.

Ce procédé est mis en oeuvre simplement et est approprié au consommateur. On fait passer un fluide sous pression, dont la température est de préférence supérieure ou égale à 30 °C, pendant un laps de temps très court, à travers au moins une substance végétale non colorante sous forme solide ou pâteuse, de préférence solide, et encore plus préférentiellement pulvérulente.

Il permet d'utiliser des substances végétales non colorantes, fraîches ou séchées, d'obtenir des compositions aqueuses concentrées en agents de traitement provenant de ces substances végétales non colorantes, et d'améliorer ainsi leur efficacité tout en évitant la dégradation desdits agents de traitement.

Les compositions préparées selon ce procédé peuvent présenter une stabilité au stockage limitée, ce qui n'est pas ici un inconvénient puisque le procédé conduit à une composition prête à l'emploi, destinée à être utilisée rapidement après sa préparation, par exemple dans les 5 minutes suivant sa préparation, notamment après refroidissement à une température acceptable pour les matières kératiniques, de préférence inférieure à 60 °C, mieux inférieure à 50 °C. La composition peut être également utilisée jusqu'à une semaine après sa préparation, selon la vitesse de dégradation de l'agent de traitement.

Etant donné le temps de préparation très court, les compositions de traitement cosmétique peuvent être préparées "à la demande" en mélangeant les composés actifs, i.e. selon les propriétés cosmétiques recherchées, dans un mode particulier de réalisation.

Selon un autre mode de réalisation, les composés actifs pouvant être conditionnés dans un dispositif prêt-à-l'emploi, il n'est pas nécessaire de déterminer au préalable les concentrations des composés actifs, ce qui limite les erreurs de mesure de l'utilisateur.

En outre, le procédé selon l'invention permet d'éviter l'utilisation de flacons multi-compartiments, ce qui rend le procédé particulièrement économique et plus sûr pour l'utilisateur.

La composition ainsi obtenue peut être utilisée seule ou en mélange avec une autre composition.

L'invention a donc pour objet un procédé de préparation d'une composition de traitement cosmétique des matières kératiniques comprenant une étape de percolation d'un fluide sous une pression d'au moins 3 bars au travers d'au moins une substance végétale non colorante sous forme solide ou pâteuse.

Un autre objet de l'invention est une composition susceptible d'être obtenue par le procédé selon l'invention.

L'invention a également pour objet l'utilisation de la composition obtenue selon le procédé de l'invention, pour le traitement cosmétique des matières kératiniques telles que la peau, les lèvres, les cheveux, les cils et les ongles.

L'invention a aussi pour objet un procédé de traitement de traitement cosmétique des matières kératiniques des êtres humains, comprenant a) la préparation d'une composition cosmétique prête à l'emploi par percolation d'un fluide sous pression d'au moins 3 bars au travers d'au moins une substance végétale non colorante sous forme solide ou pâteuse, et b) l'application de la composition obtenue à l'étape a) aux matières kératiniques.

L'application aux matières kératiniques peut, selon la nature des substances végétales non colorantes, être réalisée par voie topique ou voie orale, et de préférence par voie topique.

L'invention a encore pour objet un dispositif de conditionnement permettant de mettre en oeuvre le procédé de préparation de la présente invention.

D'autres objets, caractéristiques, aspects et avantages de l'invention apparaîtront encore plus clairement à la lecture de la description et de l'exemple qui suit.

Selon l'invention, le procédé de préparation d'une composition de traitement cosmétique des matières kératiniques comprend une étape de percolation d'un fluide à une température de préférence supérieure ou égale à 30 °C, mieux encore allant de 30 à 150 °C, et encore plus préférentiellement de 40 à 120 °C, sous une pression d'au moins 3 bars (3. 10⁵ Pa) au travers d'au moins une substance végétale non colorante sous forme solide ou pâteuse.

La percolation est un mouvement de fluide à travers un milieu poreux permettant le passage du fluide, sous l'action ou l'effet de la pression.

Le fluide comprend au moins de la vapeur d'eau. Il peut être constitué par de la vapeur d'eau éventuellement accompagnée d'eau liquide, ou par un mélange de vapeur d'eau éventuellement accompagnée d'eau liquide, et d'un ou plusieurs solvants liquides et/ou gazeux cosmétiquement acceptables. De préférence, le fluide est de la vapeur d'eau pouvant être accompagnée d'eau liquide.

A titre de solvant organique, on peut par exemple citer les alcools inférieurs en C₁-C₄ tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

La substance végétale non colorante est sous forme solide ou sous forme pâteuse, de préférence sous forme solide, et encore plus préférentiellement pulvérulente.

La substance végétale non colorante peut être fraîche ou séchée et se présenter sous forme de feuilles, de racines, de tiges, d'écorces, de fleurs, de pétales, de pistils, de fruits ou de graines, ou d'extraits obtenus à partir la plante entière ou de ses différentes parties.

Par « forme pâteuse » au sens de la présente invention, on entend une consistance intermédiaire entre une phase solide et une phase liquide. La viscosité de cette phase pâteuse est de préférence supérieure à 0,1 Pa.s, et encore plus préférentiellement supérieure à 1 Pa.s, ceci à 25°C avec un taux de cisaillement de 10 s⁻¹.

Par matières kératiniques, on entend la peau, les paupières, le cuir chevelu, les lèvres, et/ou les phanères tels que les ongles et les fibres kératiniques comme les cils, les sourcils et les cheveux.

Le procédé de la présente invention peut être mis en oeuvre à partir d'un dispositif classique permettant de générer un fluide sous pression, à une température de préférence supérieure ou égale à 30 °C, mieux encore allant de 30 à 150 °C, et encore plus préférentiellement allant de 40°C à 120°C. Un tel dispositif comprend une chambre résistant à la pression, équipée d'un bloc thermique, ainsi qu'un circuit d'acheminement du fluide produit vers la substance végétale non colorante.

Selon un autre mode de réalisation, le dispositif comprend en outre un réservoir de liquide(s) ainsi qu'une pompe permettant l'acheminement du ou des liquides vers la chambre.

Le liquide contenu dans le réservoir est soit de l'eau, soit un mélange d'eau et d'un ou plusieurs solvants cosmétiquement acceptables. De préférence, le liquide est de l'eau.

Un dispositif particulièrement utile pour la mise en oeuvre du procédé de la présente invention est une machine à café du type « expresso ». De telles machines sont bien connues de la technique. Par exemple, ces machine sont décrites dans les brevets AT 168405, US 2688911, DE 32433870 et IT 1265636.

Selon un mode de réalisation particulier de l'invention, l'étape de percolation est mise en oeuvre avec un fluide à une température supérieure à 30 °C, de préférence allant de 30 à 150 °C, et encore plus préférentiellement de 40 à 120 °C, sous une pression allant de 3 à 30 bars (3.10⁵ à 3.10⁶ Pa), de préférence d'au moins 4 bars (4.10⁵ Pa), mieux encore supérieure ou égale à 10 bars (10⁶ Pa), et tout particulièrement allant de 10 à 30 bars (10⁶ à 3.10⁶ Pa).

Une composition cosmétique contenant au moins une substance végétale non colorante sous forme solide ou pâteuse, peut être utilisée directement dans le dispositif générant le fluide sous pression dans un récipient destiné à cet usage. Elle peut également être conditionnée dans un dispositif de conditionnement particulier, de type monodose, comprenant un logement fermé délimité par au moins une paroi au moins en partie perméable au fluide comprenant au moins de la vapeur d'eau, sous une pression d'au moins 3 bars. De tels dispositifs sont, par exemple, décrits dans les demandes de brevets WO 00/56629, EP 512470, WO 99/03573 ou US 5897899. Ces dispositifs de conditionnement sont en général étanches à l'air, l'humidité et/ou la lumière.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétale, par exemple, en cellulose, en métal tel que l'aluminium ou en composite. De tels dispositifs sont par exemple décrits dans les demandes de brevets WO 00/56629, EP512470, US5897899 ou WO 99/03573.

Les substances végétales non colorantes utilisables dans le procédé de l'invention sont citées, par exemple, dans l'ouvrage "Encyclopedia of commun natural ingredients used in food, drugs and cosmetics", 2^{ème} édition, Albert Y. Leung et Steven Foster, Wiley-Interscience, 1996.

Elles sont choisies parmi les substances végétales présentant des propriétés adoucissantes, des propriétés anti-inflammatoires, des propriétés antiseptiques, des propriétés antisudorales, des propriétés calmantes, des propriétés cicatrisantes, des propriétés tonifiantes, des propriétés favorisant la contention de la microcirculation sanguine et lymphatique, des propriétés texturantes, des propriétés antioxydantes, des propriétés moussantes ou émulsifiantes, des propriétés photoprotectrices, des propriétés épaississantes, absorbantes et/ou des propriétés odorantes.

A titre d'exemples de substance végétale non colorante en fonction de leurs différentes propriétés, on peut notamment citer :
pour leurs propriétés adoucissantes :
   l'abricotier, le bleuet, le bouillon blanc, la camomille romaine, la matricaire, le coquelicot, le fénugrec, la guimauve, le lin, le lis, la mauve, le souci, le sureau, le tilleul, le tussilage, le psyllium, le plantain, le cognassier, le pêcher, l'oranger, le cactus, le pommier ;
pour leurs propriétés anti-inflammatoires :
   l'aigremoine, l'aubépine, la bruyère, le chiendent, le genévrier, la guimauve, le sureau, le tilleul, le fénugrec, la gentiane, la laitue, la pensée sauvage, le plantain, la ronce, le romarin, la sauge, le tamier, le tussilage, l'immortelle, la pâquerette ;
pour leurs propriétés antiseptiques :
   l'ail, l'aigremoine, la myrtille, la bardane, le chêne, la consoude, l'eucalyptus, le genévrier, le géranium rose, le laurier, la lavande, la marjolaine, la menthe, le pin, le romarin, le santal, le serpolet, le thym, la sauge, le chèvrefeuille, l'immortelle, la pâquerette, la tanaisie ;
pour leurs propriétés antisudorales :
   la sauge, le chêne, le noyer, le pin, la prêle, le tussilage ;
pour leurs propriétés astringentes :
   l'acacia, l'achillée millefeuille, l'aigremoine, l'alchemille, l'arbousier, l'armoise, la consoude, le cyprès, le chêne, l'églantier, l'hamamélis, le mûrier noir, la myrtille, le noisetier, le noyer, l'ortie, le peuplier, le plantain, la ronce, le ratanhia, le rosier, la salicaire, le saule, la tormentille, la vigne rouge, le mélilot ;
pour leurs propriétés calmantes :
   la carotte, la pensée sauvage, le sureau, le tilleul, le passiflore, le basilic, le camphre, le poirier, le pommier, la vigne, la laitue, le rosier, le gingembre ;
pour leurs propriétés cicatrisantes :
   le millepertuis, la potentille anserine, le souci, la matricaire, la camomille romaine, la consoude, l'achillée millefeuille, l'absinthe, l'aigremoine, l'armoise, l'arnica, le cerfeuil, la myrte, la pervenche, le plantain, le peuplier, la primevère, la sauge, le seneçon, le sureau, la verveine, l'angélique, l'aristoloche, l'aulne, l'aurone, la bistorte, le bouleau, le chardon béni, le genévrier, le nêflier, l'eucalyptus, la vulnéraire, la benoite, la centaurée, le chou, la joubarbe, le fraisier, la prêle, la reine des prés, le tussilage, la pensée sauvage, la bardane, la pâquerette, le lis ;
pour leurs propriétés tonifiantes :
   le millepertuis, l'églantine, le gui, le maté, le cassier, l'absinthe, l'arnica, le calament, la cannelle, le géranium, l'hysope, la marjolaine, la mélisse, le persil, le pin sylvestre, le romarin, la sarriette, le serpolet, le basilic, l'églantier, la gentiane, le houblon, le laurier blanc, la menthe, la sauge, la tormentille, l'achillée millefeuille, l'aigremoine, la benoite, la bistorte, le chêne, le cognassier, le cyprès, le marron d'Inde, le nêflier, le noyer, l'ortie, le plantain, la poire d'eau, le quintefeuille, la renouée, la salicaire, la véronique, l'angélique, l'armoise, l'aspérule, le genévrier, la moutarde, le quinquina, le fumeterre, la capucine, le cresson, le varech, le fragon, la tanaisie ;
pour leurs propriétés favorisant la contention de la microcirculation sanguine et lymphatique :
   le cassis, la myrtille et les pépins de raisin ;
pour leurs propriétés texturantes :
   le blé, le fucus ;
pour leurs propriétés antioxydantes :
   le riz, le romarin, la sauge , le thym, le thé vert, le réglisse ;
pour leurs propriétés moussantes ou émulsifiantes :
   la saponaire, le lierre, le fragon, le bois de panama, le quillaja, la salsepareille, le quino, le soja ;
pour leurs propriétés photoprotectrices :
   l'aloès, le tournesol, la réglisse, le magnolia, le kaemperia ;
pour leurs propriétés épaississantes ou absorbantes :
   le pois, le blé, les pommes de terre, le maïs ; et
pour leurs propriétés odorantes :
   le romarin, la violette, la lavande, et la rose.

De préférence, les substances végétales présentant des propriétés adoucissantes sont choisies parmi le bleuet, le coquelicot, le fénugrec, la guimauve, le lin, la mauve, le sureau et le cactus ; les substances végétales ayant des propriétés anti-inflammatoires sont choisies parmi le chiendent, la guimauve, le genévrier, la laitue, le sauge, le sureau, le romarin et l'immortelle ; les substances végétales ayant des propriétés antiseptiques sont choisies parmi la bardane, la consoude, l'aigremoine, le genévrier, le romarin et le chêne ; les substances végétales ayant des propriétés antisudorales sont choisies parmi la sauge, le pin, la prêle et le chêne ; les substances végétales ayant des propriétés astringentes sont choisies parmi l'acacia, l'hamamélis, le tormentille, l'églantier, le ratanhia et la consoude ; les substances végétales ayant des propriétés calmantes sont choisies parmi le rosier, la passiflore, la carotte, la pensée sauvage et le gingembre ; les substances végétales ayant des propriétés cicatrisantes sont choisies parmi l'arnica, la reine des prés, la sauge, la consoude, le sureau, la prêle, la bistorte, la matricaire et le lis ; les substances végétales ayant des propriétés tonifiantes sont choisies parmi le gui, le maté, le cassier, le varech, la sauge, la bistorte, le marron d'inde, le romarin et l'angélique ; la substance végétale ayant des propriétés texturantes est le blé ; les substances végétales ayant des propriétés antioxydantes sont choisies parmi le romarin et le thym ; les substances végétales ayant des propriétés moussantes ou émulsifiantes sont choisies parmi le saponaire, le bois de panama et la salsepareille ; la substance végétale ayant des propriétés photoprotectrices est l'aloès ; les substances végétales ayant des propriétés épaississantes ou absorbantes sont choisies parmi les pommes de terre et le maïs ; et les substances végétales ayant des propriétés odorantes sont choisies parmi le romarin, la lavande et la rose.

La ou les substances végétales non colorantes selon l'invention peuvent être mises en oeuvre dans le procédé de l'invention, en mélange avec un ou plusieurs adjuvants solides ou pâteux, et de préférence pulvérulents. Les adjuvants peuvent être choisis parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides comme le glucose, le saccharose, le sorbitol ou le fructose, l'oxyde de zinc, de zirconium, les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, le polyacrylamide, l'hydroxyapatite poreux, la soie, le collagène, la sciure de bois, la poudre de fucus, les farines ou extraits de blé, de riz, de pois, de lupin, de soja ou d'orge, la polyvinylpyrrolidone réticulée, l'alginate de calcium, le charbon actif, les particules de poly(chlorure de vinylidène/acrylonitrile), notamment celles commercialisées sous la dénomination générale d'« Expancel® » par la société AKZO NOBEL sous les références particulières « Expancel® WE» ou « DE » Expancels, et leurs mélanges.

Lorsqu'un ou plusieurs adjuvants sont présents, la ou les substances végétales non colorantes utilisées dans l'invention sont présents de préférence en une quantité allant de 0,5 à 99% en poids, mieux encore de 1 à 80 % en poids, et encore plus préférentiellement de 2 à 60 % en poids par rapport au poids total substance(s) végétale(s) non colorante(s) et adjuvants sous forme solide ou pâteuse.

Les plantes ou extraits de plantes utilisés dans le procédé selon l'invention, peuvent être soumis, avant la percolation, à un prétraitement tel qu'une torréfaction, un cryobroyage, ou une lyophilisation.

La composition de traitement cosmétique des matières kératiniques obtenue selon le procédé de l'invention contient outre le ou les agents de traitement et le(s) composant(s) du fluide, à savoir l'eau et/ou le(s) solvant(s) cosmétiquement acceptable(s), éventuellement tout ou partie du ou des adjuvants présents dans le mélange sous forme solide ou pâteuse.

L'invention concerne également une composition susceptible d'être obtenue par le procédé selon l'invention, la composition particulièrement préférée étant exempte d'agents conservateurs.

A partir du procédé de préparation de l'invention, on obtient une composition de traitement cosmétique qui peut être appliquée directement sur les matières kératiniques, ou qui peut être mélangée à un milieu cosmétiquement acceptable ou encore au moins un additif classiquement utilisé en cosmétique pourra y être ajouté par un opérateur. On peut également mélanger au moins deux compositions obtenues par le procédé de l'invention. La composition de traitement cosmétique résultant éventuellement de mélange(s) et/ou addition(s) indiqués ci-dessus, sera dénommée ci-après composition finale de traitement cosmétique ou composition finale.

Un mode de réalisation particulier de l'invention consiste à appliquer la composition obtenue par l'intermédiaire d'un dispositif ne nécessitant pas d'intervention humaine et équipé éventuellement d'un moyen de refroidissement.

Un autre mode de réalisation particulier consiste à ingérer la composition de traitement cosmétique obtenue selon le procédé de l'invention lorsque aucun problème de toxicité n'est connue dans le technique.

La quantité de l'extrait de la ou des substances végétales non colorantes présentes dans la composition finale de traitement cosmétique est en général comprise dans l'intervalle allant de 0,001 à 50 % en poids environ, de préférence de 0,005 à 30 % en poids, et encore plus préférentiellement de 0,01 à 20% en poids par rapport au poids total de la composition finale de traitement cosmétique.

Dans le cas où la composition cosmétique obtenue par le procédé de la présente invention est mélangée à un milieu cosmétiquement acceptable, un tel milieu est généralement constitué par de l'eau ou par un mélange d'eau et d'au moins un solvant organique pour solubiliser les composés qui ne seraient pas suffisamment solubles dans l'eau.

Par "cosmétiquement acceptable", on entend un milieu compatible avec les matières kératiniques et notamment la peau, les lèvres et/ou les phanères, et qui en outre, présente un aspect, un toucher, une odeur et éventuellement un goût agréable pour l'utilisateur.

A titre de solvant organique, on peut citer, par exemple, les alcools inférieurs en C₁-C₄ tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence allant de 1 à 40% en poids, et encore plus préférentiellement de 5 à 30% en poids par rapport au poids total de la composition finale de traitement cosmétique.

Au moins un additif classiquement utilisé en cosmétique peut être également ajouté dans les compositions de traitement cosmétique obtenues selon le procédé de la présente invention. A titre d'exemples de tels additifs, on peut citer des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement, des agents filmogènes, des céramides, des agents conservateurs, des agents opacifiants et des pigments colorés ou nacrés.

Les additifs ci-dessus sont en général présents en quantité comprise pour chacun d'eux entre 0,01 et 20% en poids par rapport au poids de la composition finale.

Bien entendu, l'homme de l'art veillera à choisir ce ou ces éventuels composés de manière telle que les propriétés avantageuses attachées intrinsèquement à la composition cosmétique conforme à l'invention ne soient pas, ou substantiellement pas, altérées par la ou les adjonctions envisagées.

Le pH de la composition finale est généralement compris entre 3 et 12, et de préférence entre 5 et 11. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les sels alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 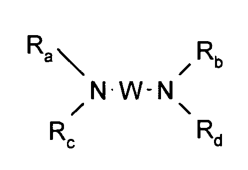 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition finale de traitement cosmétique peut se présenter sous des formes diverses, telles que sous forme de liquides, de crèmes, de gels ou sous toute autre forme appropriée pour réaliser un traitement et/ou le soin des matières kératiniques, et en particulier des fibres kératiniques, de la peau ou du cuir chevelu, ou le maquillage de la peau, des ongles, des cils ou encore des lèvres.

Elle peut être notamment utilisée pour le traitement des matières kératiniques telles que la peau et les fibres kératiniques, par exemple pour tonifier les fibres kératiniques et le cuir chevelu.

La composition finale de traitement cosmétique peut être utilisée, par exemple, comme shampoing, après-shampoing, soin rincé ou non rincé, masque de soin profond, gel douche, lotion ou crème de traitement des matières kératiniques.

La présente invention concerne aussi un procédé de traitement cosmétique des matières kératiniques, comprenant la préparation d'une composition de traitement cosmétique selon le procédé tel que défini ci-dessus, et son application sur les matières kératiniques, par exemple par l'intermédiaire d'un opérateur ou par l'intermédiaire d'un dispositif ne nécessitant pas d'intervention humaine. La durée d'application peut varier entre 15 secondes et 1 heure.

Avant application, la composition de traitement cosmétique obtenue selon le procédé de l'invention, peut être mélangée à un milieu cosmétiquement acceptable et/ou à un ou plusieurs additifs classiquement utilisés en cosmétique tels que décrits ci-dessus.

Un autre mode de réalisation consiste à préparer au moins deux compositions de traitement cosmétique selon le procédé de l'invention, à les mélanger, et à ajouter éventuellement un milieu cosmétiquement acceptable et/ou un ou plusieurs additifs classiquement utilisés en cosmétique tels que décrits ci-dessus, puis à appliquer la composition finale obtenue sur les matières kératiniques.

L'exemple ci-après est destiné à illustrer la présente invention.

### Exemple

On mélange les ingrédients solides suivants dans les proportions indiquées en % en poids par rapport au poids total de mélange solide :
- Saponines totales de lierre grimpant vendu sous la dénomination commerciale "Lierre saponines totales 50 %" par la société INDENA 50 %
- Sulfate de magnésium 50 %

On place 5 g. de ce mélange dans une machine expresso du commerce. On fait ensuite passer de la vapeur d'eau jusqu'à l'obtention d'une composition (A) présentant un volume final de 50 ml.

On obtient ainsi une composition de traitement cosmétique prête à être appliquée sur les cheveux et le cuir chevelu. Cette composition a un effet tonifiant sur la fibre et le cuir chevelu.

On peut ajouter deux parties en poids de composition (A), à une partie en poids d'une composition aqueuse (B) contenant 1 % en poids d'hydroxyéthylcellulose pour faciliter l'application.

## Revendications

1. Procédé de préparation d'une composition pour le traitement cosmétique des matières kératiniques, **caractérisé en ce qu'**il comprend une étape de percolation de fluide comprenant au moins de la vapeur d'eau, sous une pression d'au moins 3 bars au travers d'au moins une substance végétale non colorante sous forme solide ou pâteuse, choisie parmi les substances végétales présentant des propriétés adoucissantes, des propriétés anti-inflammatoires, des propriétés antiseptiques, des propriétés antisudorales, des propriétés calmantes, des propriétés cicatrisantes, des propriétés tonifiantes, des propriétés favorisant la contention de la microcirculation sanguine et lymphatique, des propriétés texturantes, des propriétés antioxydantes, des propriétés moussantes ou émulsifiantes, des propriétés photoprotectrices, des propriétés épaississantes, absorbantes et/ou des propriétés odorantes.

2. Procédé selon la revendication 1, **caractérisé en ce que** le fluide est constitué par de la vapeur d'eau éventuellement accompagnée d'eau liquide, ou par un mélange de vapeur d'eau éventuellement accompagnée d'eau liquide, et d'un ou plusieurs solvants liquides et/ou gazeux cosmétiquement acceptables.

3. Procédé selon la revendication 1 ou 2, **caractérisé en ce que** les substances végétales ayant des propriétés adoucissantes sont choisies parmi le bleuet, le coquelicot, le fénugrec, la guimauve, le lin, la mauve, le sureau et le cactus.

4. Procédé selon l'une quelconque des revendications précédentes, **caractérisé en ce que** les substances végétales ayant des propriétés anti-inflammatoires sont choisies parmi le chiendent, la guimauve, le genévrier, la laitue, la sauge, le sureau, le romarin et l'immortelle.

5. Procédé selon l'une quelconque des revendications précédentes, **caractérisé en ce que** les substances végétales ayant des propriétés antiseptiques sont choisies parmi la bardane, la consoude, l'aigremoine, le genévrier, le romarin et le chêne.

6. Procédé selon l'une quelconque des revendications précédentes, **caractérisé en ce que** les substances végétales ayant des propriétés antisudorales sont choisies parmi la sauge, le pin, la prêle et le chêne.

7. Procédé selon l'une quelconque des revendications précédentes, **caractérisé en ce que** les substances végétales ayant des propriétés astringentes sont choisies parmi l'acacia, l'hamamélis, le tormentille, l'églantier, le ratanhia et la consoude.

8. Procédé selon l'une quelconque des revendications précédentes, **caractérisé en ce que** les substances végétales ayant des propriétés calmantes sont choisies parmi le rosier, la passiflore, la carotte, la pensée sauvage et le gingembre.

9. Procédé selon l'une quelconque des revendications précédentes, **caractérisé en ce que** les substances végétales ayant des propriétés cicatrisantes sont choisies parmi l'arnica, la reine des prés, la sauge, la consoude, le sureau, la prêle, la bistorte, la matricaire et le lis.

10. Procédé selon l'une quelconque des revendications précédentes, **caractérisé en ce que** les substances végétales ayant des propriétés tonifiantes sont choisies parmi le gui, le maté, le cassier, le varech, la sauge, la bistorte, le marron d'inde, le romarin et l'angélique.

11. Procédé selon l'une quelconque des revendications précédentes, **caractérisé en ce que** les substances végétales ayant des propriétés favorisant la contention de la microcirculation sanguine et lymphatique, sont choisies parmi le cassis, la myrtille et les pépins de raisin.

12. Procédé selon l'une quelconque des revendications précédentes, **caractérisé en ce que** la substance végétale ayant des propriétés texturantes est le blé.

13. Procédé selon l'une quelconque des revendications précédentes, **caractérisé en ce que** les substances végétales ayant des propriétés antioxydantes sont choisies parmi le romarin et le thym.

14. Procédé selon l'une quelconque des revendications précédentes, **caractérisé en ce que** les substances végétales ayant des propriétés moussantes ou émulsifiantes ont choisies parmi le saponaire, le bois de panama et la salsepareille.

15. Procédé selon l'une quelconque des revendications précédentes, **caractérisé en ce que** la substance végétale ayant des propriétés photoprotectrices est l'aloès.

16. Procédé selon l'une quelconque des revendications précédentes, **caractérisé en ce que** les substances végétales ayant des propriétés épaississantes ou absorbantes sont choisies parmi les pommes de terre et le maïs.

17. Procédé selon l'une quelconque des revendications précédentes, **caractérisé en ce que** les substances végétales ayant des propriétés odorantes sont choisies parmi le romarin, la lavande et la rose.

18. Procédé selon l'une quelconque des revendications précédentes, **caractérisé en ce que** dans lequel la substance végétale non colorante sous forme solide ou pâteuse est mélangé avec un adjuvant.

19. Procédé selon la revendication 18, **caractérisé en ce que** l'adjuvant est choisi parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides, l'oxyde de zinc, de zirconium, les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, le polyacrylamide, l'hydroxyapatite poreux, la soie, le collagène, la sciure de bois, la poudre de fucus, les farines ou extraits de blé, de riz, de pois, de lupin, de soja ou d'orge, la polyvinylpyrrolidone réticulée, l'alginate de calcium, le charbon actif, et les particules de poly(chlorure de vinylidène/acrylonitrile).

20. Procédé selon la revendication 18 ou 19, **caractérisé en ce que** la substance végétale non colorante est présente en une quantité allant de 0,5 à 99% en poids, de préférence de 1 à 80 % en poids par rapport au poids total substance(s) végétale(s) non colorante(s) et adjuvant sous forme solide ou pâteuse.

21. Procédé selon l'une quelconque des revendications 18 à 20,
**caractérisé en ce que** la composition de traitement cosmétique obtenue contient outre le ou les agents de traitement et le(s) composant(s) du fluide, éventuellement tout ou partie du ou des adjuvants présents dans le mélange sous forme solide ou pâteuse.

22. Procédé selon l'une quelconque des revendications précédentes, **caractérisé en ce que** l'étape de percolation est mise en oeuvre avec un fluide sous une pression de 3 à 30 bars.

23. Procédé selon la revendication 22, **caractérisé en ce que** l'étape de percolation est mise en oeuvre avec un fluide sous une pression d'au moins 10 bars.

24. Procédé selon l'une quelconque des revendications précédentes, **caractérisé en ce que** les plantes ou extraits de plantes, sont soumis, avant la percolation, à un prétraitement.

25. Procédé selon la revendication 24, **caractérisé en ce que** le prétraitement est une torréfaction, un cryobroyage, ou une lyophilisation.

26. Composition de traitement cosmétique susceptible d'être obtenue par le procédé selon l'une quelconque des revendications précédentes.

27. Composition de traitement cosmétique selon la revendication 26, ne comprenant pas de conservateur.

28. Procédé de traitement cosmétique des matières kératiniques,
**caractérisé en ce que** l'on prépare une composition cosmétique selon le procédé défini selon l'une quelconque des revendications 1 à 25 et qu'on applique cette composition sur la matière kératinique.

29. Procédé de traitement cosmétique des matières kératiniques selon la revendication 28, **caractérisé en ce que** l'on applique cette composition sur les matières kératiniques par l'intermédiaire d'un dispositif ne nécessitant pas d'intervention humaine.

30. Procédé de traitement cosmétique des matières kératiniques,
**caractérisé en ce que** l'on prépare au moins deux compositions cosmétiques selon le procédé défini selon l'une quelconque des revendications 1 à 25, on les mélange et on applique le mélange sur les matières kératiniques.

31. Utilisation d'une composition obtenue par le procédé selon l'une quelconque des revendications 1 à 25 pour le traitement cosmétique des matières kératiniques.

32. Utilisation selon la revendication 31 pour le traitement cosmétique de la peau, des lèvres, des cheveux, des cils ou des ongles.

33. Dispositif de conditionnement d'une composition cosmétique, comprenant un logement fermé délimité par au moins une paroi au moins en partie perméable au fluide comprenant au moins de la vapeur d'eau, sous pression d'au moins 3 bars, ladite composition contenant au moins une substance végétale non colorante sous forme solide ou pâteuse, choisie parmi les substances végétales présentant des propriétés adoucissantes, des propriétés anti-inflammatoires, des propriétés antiseptiques, des propriétés antisudorales, des propriétés calmantes, des propriétés cicatrisantes, des propriétés tonifiantes, des propriétés favorisant la contention de la microcirculation sanguine et lymphatique, des propriétés texturantes, des propriétés antioxydantes, des propriétés moussantes ou émulsifiantes, des propriétés photoprotectrices, des propriétés épaississantes, absorbantes et/ou des propriétés odorantes.

34. Dispositif selon la revendication 33, **caractérisé en ce que** le logement est délimité par deux feuilles scellées.

35. Dispositif selon la revendication 33, **caractérisé en ce que** le logement est délimité par une barquette fermée par un couvercle.
